# EUROPEAN PATENT SPECIFICATION

(11) **EP 1 462 522 B1**
(45) Date of publication and mention of the grant of the patent: **16.08.2006**
(21) Application number: 04014323.2
(22) Date of filing: 25.02.1999
(51) Int. Cl.: C12N 15/29, C12N 15/53, C12N 15/82, C12N 5/10, A01H 5/00, A01H 5/10

(54) **Maize Met-1 promoter**
Mais met-1 Promoter
promoteur met-1 du mais

(30) Priority: 26.02.1998 US 76075 P
(43) Date of publication of application: 29.09.2004
(62) Divisional of application: 99909638.1
(73) Proprietor: PIONEER HI-BRED INTERNATIONAL, INC., Des Moines, Iowa 50309 (US)
(72) Inventor: Rice, Douglas, Des Moines, IA 50312 (US)
(74) Representative: Bentham, Andrew

(56) References cited:
- EP-A- 0 342 926
- EP-A- 0 353 908
- EP-A- 0 452 269
- EP-A- 0 652 286
- WO-A-90/02172
- WO-A-95/35386
- WO-A-96/07746
- WO-A-97/05260
- US-A- 5 639 952
- BECKER T. ET AL.: "The cab-m7 gene: a light-inducible, mesophyll-specific gene of maize" PLANT MOLECULAR BIOLOGY, vol. 20, 1992, pages 49-60, XP002123961
- JOANIN P ET AL: "Nucleotide sequence and expression of two cDNA coding for two histone H2B variants of maize." PLANT MOLECULAR BIOLOGY, (1992 NOV) 20 (4) 581-8. JOURNAL CODE: A6O. ISSN: 0167-4412., 1992, pages 581-588, XP002103879 Netherlands
- JOANIN P. ET AL.: "Molecular cloning and sequence analysis of 2 genes encoding 2 histone H2B variants of maize" PLANT PHYSIOLOGY AND BIOCHEMISTRY, vol. 32, no. 5, 1994, pages 693-696, XP002103880
- BRIGNON P ET AL: "Nuclease sensitivity and functional analysis of a maize histone H3 gene promoter." PLANT MOLECULAR BIOLOGY, (1993 SEP) 22 (6) 1007-15. JOURNAL CODE: A6O. ISSN: 0167-4412., vol. 32, no. 5, 1994, pages 693-696, XP002103881 Netherlands
- BRIGNON P ET AL: "Constitutive and cell-division-inducible protein-DNA interactions in two maize histone gene promoters." PLANT JOURNAL, (1993 SEP) 4 (3) 445-57. JOURNAL CODE: BRU. ISSN: 0960-7412., vol. 22, 1993, pages 1007-1015, XP002103882 ENGLAND: United Kingdom
- MANJUNATH S. AND SACHS M.: "Molecular characterization and promoter analysis of the maize cytosolic glyceraldehyde 3-phosphate dehydrogenase gene family and its expression during anoxia" PLANT MOLECULAR BIOLOGY, vol. 33, no. 1, January 1997 (1997-01), pages 97-112, XP002103883
- KYOZUKA J ET AL: "Anaerobic induction and tissue-specific expression of maize Adh1 promoter in transgenic rice plants and their progeny." MOLECULAR AND GENERAL GENETICS, (1991 AUG) 228 (1-2) 40-8. JOURNAL CODE: NGP. ISSN: 0026-8925., vol. 228, 1991, pages 40-48, XP002079665 GERMANY: Germany, Federal Republic of
- MARKMANN-MULISCH U ET AL: "Nucleotide sequence and linkage map position of the genes for ribosomal proteins L14 and S8 in the maize chloroplast genome." EUROPEAN JOURNAL OF BIOCHEMISTRY, (1988 JAN 4) 170 (3) 507-14., vol. 170, 1988, pages 507-514, XP000856629

## Description

### FIELD OF THE INVENTION

The present invention relates to the field of plant molecular biology, more particularly to regulation of gene expression in plants.

### BACKGROUND OF THE INVENTION

Expression of heterologous DNA sequences in a plant host is dependent upon the presence of an operably linked promoter that is functional within the plant host. Choice of the promoter sequence will determine when and where within the organism the heterologous DNA sequence is expressed. Thus, where continuous expression is desired throughout the cells of a plant, constitutive promoters are utilized. In contrast, where gene expression in response to a stimulus is desired, inducible promoters are the regulatory element of choice. In either case, additional regulatory sequences upstream and/or downstream from the core promoter sequence may be included in expression constructs of transformation vectors to bring about varying levels of constitutive or inducible expression of heterologous nucleotide sequences in a transgenic plant.

Frequently it is desirable to have constitutive expression of a DNA sequence throughout the cells of an organism. For example, increased resistance of a plant to infection by soil- and air-borne pathogens might be accomplished by genetic manipulation of the plant's genome to comprise a constitutive promoter operably linked to a heterologous pathogen-resistance gene such that pathogen-resistance proteins are continuously expressed throughout the plant's tissues.

Alternatively, it might be desirable to inhibit expression of a native DNA sequence within a plant's tissues to achieve a desired phenotype. In this case, such inhibition might be accomplished with transformation of the plant to comprise a constitutive promoter operably linked to an antisense nucleotide sequence, such that constitutive expression of the antisense sequence produces an RNA transcript that interferes with translation of the mRNA of the native DNA sequence.

Thus, isolation and characterization of constitutive promoters that can serve as regulatory regions for constitutive expression of heterologous nucleotide sequences of interest are needed for genetic manipulation of plants to exhibit specific phenotypic traits.

### SUMMARY OF THE INVENTION

Compositions and methods for regulating expression of heterologous nucleotide sequences in a plant are provided.

The invention therefore provides an isolated nucleic acid molecule having a nucleotide sequence for a promoter that is capable of initiating transcription in a plant cell, wherein said nucleotide sequence is selected from:
(a) a nucleotide sequence comprising the sequence set forth in SEQ ID NO: 1;
(b) a nucleotide sequence deposited as ATCC Accession No. 207120;
(c) a nucleotide sequence comprising at least 40 contiguous nucleotides of
the sequence set forth in SEQ ID NO: 1;
(d) a nucleotide sequence that hybridizes under stringent conditions to a sequence of a), b) or c); and
(e) a nucleotide sequence that has at least 70% sequence identity to a sequence set forth in a) or b).

The invention also provides a DNA construct comprising a nucleotide sequence of the invention operably linked to a heterologous nucleotide sequence of interest.

The invention also provides a vector comprising the DNA construct according to the invention.

The invention also provides a host cell having stably incorporated in its genome the DNA construct of the invention.

A composition comprising a novel nucleotide sequence for a constitutive plant promoter isolated from the maize gene encoding a metallothionein is provided.

A method for constitutively expressing a heterologous nucleotide sequence in a plant using the promoter sequence disclosed herein is also provided. The method comprises transforming a plant cell with a transformation vector that comprises a heterologous nucleotide sequence operably linked to the plant promoter of the present invention and regenerating a stably transformed plant from the transformed plant cell.

The invention also provides a plant cell or plant stably transformed with a DNA construct according to the invention and a seed of such plant.

### BRIEF DESCRIPTION OF THE DRAWINGS

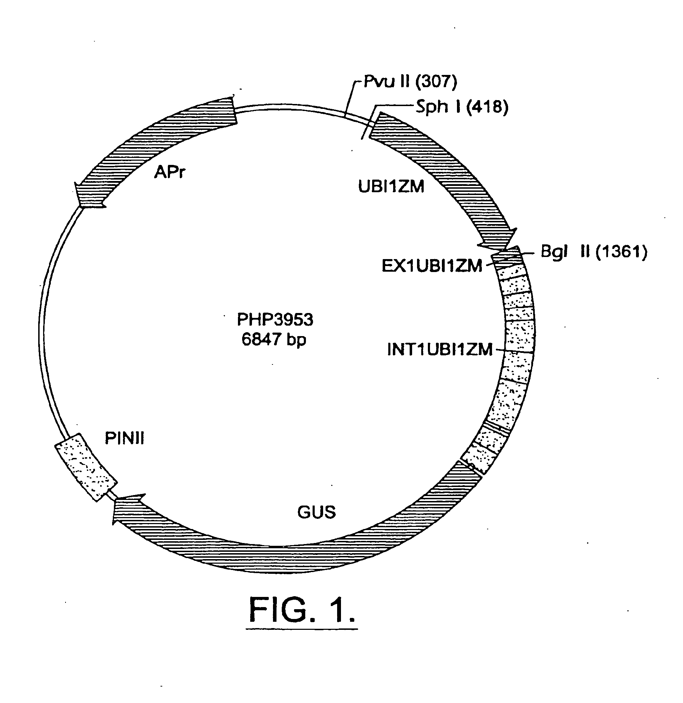

Figure 1 shows the plasmid vector PHP3953 comprising the GUS gene operably linked to the ubiquitin promoter. Promoter fragments of the present invention were recloned into this plasmid in place of the ubiquitin promoter, and the resulting plasmid DNA was available for use in transformation studies to test for promoter activity.

### DETAILED DESCRIPTION OF THE INVENTION

The composition of the present invention is a nucleic acid molecule comprising a novel nucleotide sequence for a plant promoter, more particularly the constitutive promoter for the maize gene encoding metallothionein-1. The nucleotide sequence for this promoter is set forth in SEQ ID NO: 1. In particular, the present invention provides for an isolated nucleic acid molecule comprising a nucleotide sequence encoding the DNA sequence deposited in a bacterial host as ATCC Accession No. 207120, and variants and fragments thereof. The promoter for this maize gene was isolated from the 5' untranslated region flanking its respective transcription initiation site. Methods for isolation of promoter regions are well known in the art. The specific method used to obtain the promoter of the present invention is described in Example 1 below.

A plasmid containing the promoter nucleotide sequence of the invention has been deposited with American Type Culture Collection (ATCC), Manassas, Virginia, and assigned Accession No. 207120. This deposit will be maintained under the terms of the Budapest Treaty on the International Recognition of the Deposit of Microorganisms for the Purposes of Patent Procedure.

The invention encompasses an isolated or substantially purified nucleic acid composition. An "isolated" or "purified" nucleic acid molecule, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived.

By "promoter" is intended a regulatory region of DNA usually comprising a TATA box capable of directing RNA polymerase II to initiate RNA synthesis at the appropriate transcription initiation site for a particular coding sequence. A promoter may additionally comprise other recognition sequences generally positioned upstream or 5' to the TATA box, referred to as upstream promoter elements, which influence the transcription initiation rate. It is recognized that having identified the nucleotide sequence for the promoter region disclosed herein, it is within the state of the art to isolate and identify further regulatory elements in the 5' untranslated region upstream from the particular promoter region identified herein. Thus the promoter region disclosed herein may further comprise upstream regulatory elements that confer tissue-specific expression of any heterologous nucleotide sequence operably linked to the disclosed promoter sequence. See particularly Australian Patent No. AU-A-77751/94 and U.S. Patent Nos. 5,466,785 and 5,635,618.

The maize promoter sequence of the present invention, when assembled within a DNA construct such that the promoter is operably linked to a heterologous nucleotide sequence of interest, enables constitutive expression of the heterologous nucleotide sequence in the cells of a plant stably transformed with this DNA construct. By "heterologous nucleotide sequence" is intended a sequence that is not naturally occurring with the promoter sequence. While this nucleotide sequence is heterologous to the promoter sequence, it may be homologous or native or heterologous or foreign to the plant host. By "constitutive" is intended expression in the cells throughout a plant at most times and in most tissues.

The isolated promoter sequence of the present invention can be classified as a weak constitutive promoter. Generally, by "weak promoter" is intended a promoter that drives expression of a coding sequence at a low level. By "low level" is intended at levels of about 1/10,000 transcripts to about 1/100,000 transcripts to about 1/500,000 transcripts. Conversely, a strong promoter drives expression of a coding sequence at a high level, or at about 1/10 transcripts to about 1/100 transcripts to about 1/1,000 transcripts.

The nucleotide sequence for the constitutive promoter of the present invention may be the naturally occurring sequence or any sequence having substantial homology. By "substantial homology" is intended a sequence exhibiting substantial functional and structural equivalence with the native or naturally occurring sequence. Any functional or structural differences between substantially homologous sequences do not affect the ability of the sequence to function as a promoter as disclosed in the present invention. Thus, any sequence having substantial sequence homology with the sequence of the constitutive promoter of the present invention will direct constitutive expression of an operably linked heterologous nucleotide sequence. Two promoter nucleotide sequences are considered substantially homologous when they have at least about 50%, 60%, to 70%, generally about 80%, preferably about 85%, 90%, up to 98% sequence homology.

Substantially homologous sequences of the present invention include variants of the disclosed sequences, such as those that result from site-directed mutagenesis, as well as synthetically derived sequences. Methods for mutagenesis and nucleotide sequence alterations are well known in the art. See, for example, Kunkel (1985) *Proc. Natl. Acad Sci. USA* 82:488-492; Kunkel *et al.* (1987) *Methods Enzymol. 154*:367-382; U.S. Patent No. 4,873,192; Walker *et al.,* eds. (1983) *Techniques in Molecular Biology* (MacMillan Publishing Company, New York) and the references cited therein. Thus, the promoter nucleotide sequence of the invention includes both the naturally occurring sequence as well as mutant and synthetically derived forms. Generally, nucleotide sequence variants of the invention will have at least about 50%, 60%, to 70%, generally about 80%, preferably about 85%, 90%, up to 98% sequence identity to its respective native nucleotide sequence.

Fragments of the promoter nucleotide sequence disclosed herein are also encompassed by the present invention. By "fragment" is intended a portion of the promoter nucleotide sequence. Fragments of a promoter nucleotide sequence may retain their biological activity. Thus, for example, less than the entire promoter sequence disclosed herein may be utilized to drive expression of an operably linked nucleotide sequence of interest, such as a nucleotide sequence encoding a heterologous protein. It is within skill in the art to determine whether such fragments decrease expression levels or alter the nature of expression, *i.e.*, constitutive expression. Alternatively, fragments of a promoter nucleotide sequence that are useful as hybridization probes generally do not retain this biological activity.

Nucleic acid molecules that are fragments of a promoter nucleotide sequence comprise at least 40, 45, 50, 75, 100, 325, 350, 375, 400, 425, 450, 500, 550, 600, 650, 700, 800, 900, 1,000, 1,100, 1,200 or 1,300 nucleotides, or up to the number of nucleotides present in a full-length promoter nucleotide sequence disclosed herein (*i.e.,* 1300 for SEQ ID NO: 1). Generally, fragments of a promoter sequence that retain their biological activity comprise at least 40 contiguous nucleotides, preferably at least 50 contiguous nucleotides, more preferably at least 75 contiguous nucleotides, still more preferably at least 100 contiguous nucleotides of the promoter nucleotide sequence disclosed herein. Preferred fragment lengths depend upon the objective and will also vary depending upon the promoter sequence.

The nucleotide sequences of such fragments will usually comprise the TATA recognition sequence of the promoter sequence. Such fragments may be obtained by use of restriction enzymes to cleave the naturally occurring promoter nucleotide sequence disclosed herein; by synthesizing a nucleotide sequence from the naturally occurring sequence of the promoter DNA sequence; or may be obtained through the use of PCR technology. See particularly, Mullis *et al.* (1987) *Methods Enzymol.* 155:335-350, and Erlich, ed. (1989) *PCR Technology* (Stockton Press, New York). Variants of these promoter fragments, such as those resulting from site-directed mutagenesis, are encompassed by the compositions of the present invention.

The disclosed promoter nucleotide sequence can be used to isolate homologous promoter sequences in other plant species. Methods are readily available in the art for the hybridization of nucleic acid sequences. Promoter sequences from other plants may be isolated according to well-known techniques based on their sequence homology to the promoter sequence set forth herein. In these techniques all or part of the known nucleotide sequence is used as a probe which selectively hybridizes to other promoter nucleotide sequences present in a population of cloned genomic DNA fragments or cDNA fragments (i.e., genomic or cDNA libraries) from a chosen organism.

To obtain other homologous promoter sequences, the entire promoter nucleotide sequence or portions thereof may be used as probes capable of specifically hybridizing to corresponding promoter sequences. To achieve specific hybridization under a variety of conditions, such probes include sequences that are unique and are preferably at least about 10 nucleotides in length, and most preferably at least about 20 nucleotides in length. Such probes may be used to amplify the promoter sequences of interest from a chosen organism by the well-known process of polymerase chain reaction (PCR). This technique may be used to isolate additional promoter sequences from a desired organism or as a diagnostic assay to determine the presence of promoter sequences in an organism.

Such techniques include hybridization screening of plated DNA libraries (either plaques or colonies; see, *e.g.,* Sambrook *et al.* (1989) *Molecular Cloning: A Laboratory Manual* (2d ed., Cold Spring Harbor Laboratory Press, Plainview, New York) and amplification by PCR using oligonucleotide primers (see, *e.g.,* Innis *et al., eds.* (1990) *PCR Protocols, a Guide to Methods and Applications,* Academic Press, New York).

For example, hybridization of such sequences may be carried out under conditions of reduced stringency, medium stringency, or even stringent conditions (e.g., conditions represented by a wash stringency of 35-40% Formamide with 5x Denhardt's solution, 0.5% SDS and 1x SSPE at 37°C; conditions represented by a wash stringency of 40-45% Formamide with 5x Denhardt's solution, 0.5% SDS, and 1xSSPE at 42°C; and conditions represented by a wash stringency of 50% Formamide with 5x Denhardt's solution, 0.5% SDS and 1x SSPE at 42°C, respectively) to DNA for the promoter sequence disclosed herein in a standard hybridization assay. See Sambrook *et al.* (1989) *Molecular Cloning: A Laboratory Manual* (2d ed., Cold Spring Harbor Laboratory Press, Plainview, New York). In general, homologous sequences that are promoter sequences and hybridize to the promoter sequence disclosed herein will be at least 70% homologous, and even 80%, 85%, 90%, 95% homologous or more with the disclosed sequence. That is, the sequence similarity of sequences may range, sharing at least about 70%, and even about 80%, 85%, 90%, 95%, 98% sequence similarity.

The following terms are used to describe the sequence relationships between two or more nucleic acids: (a) "reference sequence", (b) "comparison window", (c) "sequence identity", (d) "percentage of sequence identity", and (e) "substantial identity".
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence, a gap penalty is typically introduced and is subtracted from the number of matches.
   Methods of alignment of sequences for comparison are well known in the art. Optimal alignment of sequences for comparison may be conducted by the local homology algorithm of Smith *et al.* (1981) *Adv. Appl. Math.* 2:482; by the homology alignment algorithm of Needleman *et al.* (1970) *J. Mol. Biol.* 48:443; by the search for similarity method of Pearson *et al.* (1988) *Proc. Natl. Acad. Sci.* 85:2444; by computerized implementations of these algorithms, including, but not limited to: CLUSTAL in the PC/Gene program by Intelligenetics, Mountain View, California; GAP, BESTFIT, BLAST, FASTA, and TFASTA in the Wisconsin Genetics Software Package, Genetics Computer Group (GCG), 575 Science Drive, Madison, Wisconsin; the CLUSTAL program is well described by Higgins *et al.* (1988) *Gene* 73:237-244 (1988); Higgins *et al.* (1989) *CABIOS* 5:151-153; Corpet *et al.* (1988) *Nuc. Acids Res.* 16:10881-90; Huang *et al.* (1992) *Computer Applications in the Biosciences* 8:155-65, and Person *et al.* (1994) *Meth. Mol. Biol.* 24:307-331; preferred computer alignment methods also include the BLASTP, BLASTN, and BLASTX algorithms (see Altschul *et al.* (1990) *J. Mol. Biol.* 215:403-410). Alignment is also often performed by inspection and manual alignment.
(c) As used herein, "sequence identity" or "identity" in the context of two nucleic acid sequences makes reference to the residues in the two sequences that are the same when aligned for maximum correspondence over a specified comparison window.
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison, and multiplying the result by 100 to yield the percentage of sequence identity.
(e)(i) The term "substantial identity" of polynucleotide sequences means that a polynucleotide comprises a sequence that has at least 70% sequence identity, preferably at least 80%, more preferably at least 90% and most preferably at least 95%, compared to a reference sequence using one of the alignment programs described using standard parameters.

Another indication that nucleotide sequences are substantially identical is if two molecules hybridize to each other under stringent conditions. Generally, stringent conditions are selected to be about 5°C to about 20°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Typically, stringent wash conditions are those in which the salt concentration is about 0.02 molar at pH 7 and the temperature is at least about 50, 55, or 60°C.

The nucleotide sequence for the constitutive promoter disclosed in the present invention, as well as a variant or fragment thereof, is useful in the genetic manipulation of any plant when assembled within a DNA construct such that the promoter sequence is operably linked with a heterologous nucleotide sequence whose constitutive expression is to be controlled to achieve a desired phenotypic response. By "operably linked" is intended the transcription or translation of the heterologous nucleotide sequence is under the influence of the promoter sequence. In this manner, the nucleotide sequence for the promoter of the invention is provided in expression cassettes along with heterologous nucleotide sequences for expression in the plant of interest. It is recognized that the promoter sequence of the invention may also be used with its native coding sequence to increase or decrease expression of the native coding sequence, thereby resulting in a change in phenotype in the transformed plant.

Such expression cassettes will comprise a transcriptional initiation region comprising the promoter nucleotide sequence of the present invention, or a variant or fragment thereof, operably linked to the heterologous nucleotide sequence whose expression is to be controlled by the constitutive promoter disclosed herein. Such an expression cassette is provided with a plurality of restriction sites for insertion of the nucleotide sequence to be under the transcriptional regulation of the regulatory regions. The expression cassette may additionally contain selectable marker genes.

In order to increase transcription levels, enhancers may be utilized in combination with the promoter region of the invention. Enhancers are nucleotide sequences that act to increase the expression of a promoter region. Enhancers are known in the art and include the SV40 enhancer region, the 35S enhancer element, and the like.

The transcriptional cassette will include in the 5'-to-3' direction of transcription, a transcriptional and translational initiation region, a heterologous nucleotide sequence of interest, and a transcriptional and translational termination region functional in plants. The termination region may be native with the transcriptional initiation region comprising the promoter nucleotide sequence of the present invention, may be native with the DNA sequence of interest, or may be derived from another source. Convenient termination regions are available from the Ti-plasmid of *A. tumefaciens,* such as the octopine synthase and nopaline synthase termination regions. See also, Guerineau *et al.* (1991) *Mol. Gen. Genet.* 262:141-144; Proudfoot (1991) *Cell* 64:671-674; Sanfacon *et al.* (1991) *Genes Dev.* 5:141-149; Mogen *et al.* (1990) *Plant Cell* 2:1261-1272; Munroe *et al.* (1990) *Gene* 91:151-158; Ballas *et al.* 1989) *Nuc. Acids Res.* 17:7891-7903; Joshi *et al.* (1987) *Nuc. Acid Res.* 15:9627-9639.

The expression cassette comprising the promoter sequence of the present invention operably linked to a heterologous nucleotide sequence may also contain at least one additional nucleotide sequence for a gene to be cotransformed into the organism. Alternatively, the additional sequence(s) can be provided on another expression cassette.

Where appropriate, the heterologous nucleotide sequence whose expression is to be under the control of the promoter sequence of the present invention and any additional nucleotide sequence(s) may be optimized for increased expression in the transformed plant. That is, these nucleotide sequences can be synthesized using plant- preferred codons for improved expression. Methods are available in the art for synthesizing plant-preferred nucleotide sequences. See, for example, U.S. Patent Nos. 5,380,831 and 5,436,391, and Murray *et al.* (1989) *Nuc. Acids Res.* 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the heterologous nucleotide sequence may be adjusted to levels average for a given cellular host, as calculated by reference to known genes expressed in the host cell. When possible, the sequence is modified to avoid predicted hairpin secondary mRNA structures.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) (Elroy-Stein *et al.* (1989) *Proc. Nat. Acad. Sci. USA* 86:6126-6130); potyvirus leaders, for example, TEV leader (Tobacco Etch Virus) (Allison *et al.* (1986)); MDMV leader (Maize Dwarf Mosaic Virus) *(Virology* 154:9-20); human immunoglobulin heavy-chain binding protein (BiP) (Macejak and Sarnow (1991) *Nature* 353:90-94); untranslated leader from the coat protein mRNA of alfalfa mosaic virus (AMV RNA 4) (Jobling and Gehrke (1987) *Nature* 325:622-625); tobacco mosaic virus leader (TMV) (Gallie *et al.* (1989) *Molecular Biology of RNA,* pages 237-256); and maize chlorotic mottle virus leader (MCMV) (Lommel *et al.* (1991) *Virology* 81:382-385). See also Della-Cioppa *et al.* (1987) *Plant Physiology* 84:965-968. Other methods known to enhance translation and/or mRNA stability can also be utilized, for example, introns, and the like.

In those instances where it is desirable to have the constitutively expressed product of the heterologous nucleotide sequence directed to a particular organelle, such as the chloroplast or mitochondrion, or secreted at the cell's surface or extracellularly, the expression cassette may further comprise a coding sequence for a transit peptide. Such transit peptides are well known in the art and include, but are not limited to, the transit peptide for the acyl carrier protein, the small subunit of RUBISCO, plant EPSP synthase, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, for example, transitions and transversions, may be involved.

The expression cassette comprising the promoter sequence of the present invention operably linked to a heterologous nucleotide sequence of interest can be used to transform any plant. In this manner, genetically modified plants, plant cells, plant tissue, seed, and the like can be obtained. Transformation protocols may vary depending on the type of plant or plant cell, *i.e.,* monocot or dicot, targeted for transformation. Suitable methods of transforming plant cells include microinjection (Crossway *et al.* (1986) *Biotechniques* 4:320-334), electroporation (Riggs *et al.* (1986) *Proc. Natl. Acad. Sci. USA* 83:5602-5606), Agrobacterium-mediated transformation (*Hinchee et al.* (1988) *Biotechnology* 6:915-921), direct gene transfer (Paszkowski *et al.* (1984) *EMBO J.* 3:2717-2722), and ballistic particle acceleration (see, for example, Sanford *et al.* U.S. Patent 4,945,050; Tomes *et al.* (1995) in *Plant Cell, Tissue, and Organ Culture: Fundamental Methods,* ed. Gamborg and Phillips (Springer-Verlag, Berlin); and McCabe *et al.* (1988) *Biotechnology* 6:923-926). Also see Weissinger *et al.* (1988) *Annual Rev. Genet.* 22:421-477; Sanford *et al.* (1987) *Particulate Science and Technology* 5:27-37 (onion); Christou *et al.* (1988) *Plant Physiol.* 87:671-674 (soybean); McCabe *et al.* (1988) *Bio*/*Technology* 6:923-926 (soybean); Datta *et al.* (1990) *Biotechnology* 8:736-740 (rice); Klein *et al.* (1988) *Proc. Natl. Acad. Sci. USA* 85:4305-4309 (maize); Klein *et al.* (1988) *Biotechnology* 6:559-563 (maize); Klein *et al.* (1988) *Plant Physiol.* 91:440-444 (maize); Fromm *et al.* (1990) *Biotechnology* 8:833-839; Hooydaas-Van Slogteren and Hooykaas (1984) *Nature (London)* 311:763-764; Bytebier *et al.* (1987) *Proc. Natl. Acad. Sci. USA* 84:5345-5349 (Liliaceae); De Wet *et al.* (1985) in *The Experimental Manipulation of Ovule Tissues,* ed. G. P. Chapman *et al.* (Longman, New York), pp. 197-209 (pollen); Kaeppler *et al.* (1990) *Plant Cell Reports* 9:415-418; and Kaeppler *et al.* (1992) *Theor. Appl. Genet.* 84:560-566 (whisker-mediated transformation); D'Halluin *et al.* (1992) *Plant Cell* 4:1495-1505 (electroporation); Li *et al.* (1993) *Plant Cell Reports* 12:250-255 and Christou and Ford (1995) *Annals of Botany* 75:407-413 (rice); Osjoda *et al.* (1996) *Nature Biotechnology* 14:745-750 (maize via *Agrobacterium tumefaciens*)*.*

The cells that have been transformed may be grown into plants in accordance with conventional ways. See, for example, McCormick *et al.* (1986) *Plant Cell Reports* 5:81-84. These plants may then be grown, and either pollinated with the same transformed strain or different strains, and the resulting hybrid having constitutive expression of the desired phenotypic characteristic identified. Two or more generations may be grown to ensure that constitutive expression of the desired phenotypic characteristic is stably maintained and inherited and then seeds harvested to ensure constitutive expression of the desired phenotypic characteristic has been achieved.

The promoter nucleotide sequence and methods disclosed herein are useful in regulating constitutive expression of any heterologous nucleotide sequence in a host plant in order to vary the phenotype of a plant. Various changes in phenotype are of interest including modifying the fatty acid composition in a plant, altering the amino acid content of a plant, altering a plant's pathogen defense mechanism, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in phenotype of the transformed plant.

Genes of interest are reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for transformation will change accordingly. General categories of genes of interest include for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, sterility, grain characteristics, and commercial products. Genes of interest include, generally, those involved in oil, starch, carbohydrate, or nutrient metabolism as well as those affecting kernel size, sucrose loading, and the like.

Agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Patent Application Serial Nos. 08/838,763, filed April 10, 1997, from which derives WO 94/16078, published July 21, 1994; 08/824,379, filed March 26, 1997, from which derives WO 96/38562, published December 5, 1996; 08/824,382, filed March 26, 1997, from which derives WO 96/38563, published December 5, 1996; and U.S. Patent No. 5,703,409. Another example is lysine and/or sulfur rich seed protein encoded by the soybean 2S albumin described in U.S. Serial No. 08/618,911, filed March 20, 1996, from which derives WO 97/35023, published September 25, 1997; and the chymotrypsin inhibitor from barley, Williamson *et al.* (1987) *Eur. J. Biochem.* 165:99-106.

Derivatives of the coding sequences can be made by site directed mutagenesis to increase the level of preselected amino acids in the encoded polypeptide. For example, the gene encoding the barley high lysine polypeptide (BHL) is derived from barley chymotrypsin inhibitor, PCT/US97/20441, filed October 31, 1997. Other proteins include methionine-rich plant proteins such as from sunflower seed (Lilley *et al.* (1989) *Proceedings of the World Congress on Vegetable Protein Utilization in Human Foods and Animal Feedstuffs,* ed. Applewhite (American Oil Chemists Society, Champaign, Illinois, pp. 497-502); corn (Pedersen *et al.* (1986) *J. Biol. Chem.* 261:6279; Kirihara *et al.* (1988) *Gene* 71:359); and rice (Musumura *et al.* (1989) *Plant Mol. Biol.* 12:123). Other agronomically important genes encode latex, Floury 2, growth factors, seed storage factors, and transcription factors.

Insect resistance genes may encode resistance to pests that have great yield drag such as rootworm, cutworm, European Corn Borer, and the like. Such genes include, for example *Bacillus thuringiensis* toxic protein genes (U.S. Patent Nos. 5,366,892; 5,747,450; 5,737,514; 5,723,756; 5,593,881; Geiser *et al.* (1986) *Gene* 48:109); lectins (Van Damme *et al.* (1994) *Plant Mol. Biol.* 24:825); and the like.

Genes encoding disease resistance traits include detoxification genes, such as against fumonosin (U.S. Patent Application Serial No. 08/484,815, filed June 7, 1995, from which derives WO 96/06175, published February 29, 1996); avirulence (avr) and disease resistance (R) genes (Jones *et al.* (1994) *Science* 266:789; Martin *et al.* (1993) *Science* 262:1432; Mindrinos *et al.* (1994) *Cell 78:1089);* and the like.

Herbicide resistance traits may include genes coding for resistance to herbicides that act to inhibit the action of acetolactate synthase (ALS), in particular the sulfonylurea-type herbicides (e.g., the acetolactate synthase (ALS) gene containing mutations leading to such resistance, in particular the S4 and/or Hra mutations), genes coding for resistance to herbicides that act to inhibit action of glutamine synthase, such as phosphinothricin or basta (e.g., the bar gene), or other such genes known in the art. The *bar* gene encodes resistance to the herbicide basta, the *nptII* gene encodes resistance to the antibiotics kanamycin and geneticin, and the ALS gene encodes resistance to the herbicide chlorsulfuron.

Sterility genes can also be encoded in an expression cassette and provide an alternative to physical detasseling. Examples of genes used in such ways include male tissue-preferred genes and genes with male sterility phenotypes such as QM, described in U.S. Patent No. 5,583,210. Other genes include kinases and those encoding compounds toxic to either male or female gametophytic development.

The quality of grain is reflected in traits such as levels and types of oils, saturated and unsaturated, quality and quantity of essential amino acids, and levels of cellulose. In corn, modified hordothionin proteins, described in U.S. Patent Application Serial Nos. 08/838,763, filed April 10, 1997, from which derives WO 94/16078, published July 21, 1994; 08/824,379, filed March 26, 1997, from which derives WO 96/38562, published December 5, 1996; 08/824,382, filed March 26, 1997, from which derives WO 96/38563, published December 5, 1996; and U.S. Patent No. 5,703,409 issued December 30, 1997, provide descriptions of modifications of proteins for desired purposes.

Commercial traits can also be encoded on a gene or genes that could increase for example, starch for ethanol production, or provide expression of proteins. Another important commercial use of transformed plants is the production of polymers and bioplastics such as described in U.S. Patent No. 5,602,321 issued February 11, 1997. Genes such as B-Ketothiolase, PHBase (polyhydroxyburyrate synthase) and acetoacetyl-CoA reductase (see Schubert *et al.* (1988) *J. Bacteriol.* 170:5837-5847) facilitate expression of polyhyroxyalkanoates (PHAs).

Exogenous products include plant enzymes and products as well as those from other sources including procaryotes and other eukaryotes. Such products include enzymes, cofactors, hormones, and the like. The level of proteins, particularly modified proteins having improved amino acid distribution to improve the nutrient value of the plant, can be increased. This is achieved by the expression of such proteins having enhanced amino acid content.

Thus, the heterologous nucleotide sequence operably linked to the constitutive promoter disclosed herein may be a structural gene encoding a protein of interest. Examples of such heterologous genes include, but are not limited to, genes encoding proteins conferring resistance to abiotic stress, such as drought, temperature, salinity, and toxins such as pesticides and herbicides, or to biotic stress, such as attacks by fungi, viruses, bacteria, insects, and nematodes, and development of diseases associated with these organisms. More particularly, the constitutive promoter disclosed herein and identified as a weak constitutive promoter is useful in transforming plants to constitutively express an avirulence gene as disclosed in the copending applications both entitled *"Methods for Enhancing Disease Resistance in Plants,"* U.S. Application Serial No. 60/075,151, filed February 26, 1998, and U.S. Application Serial No. 60/092,464, filed July 11, 1998, from both of which derives WO 99/43823, published September 2, 1999. Such a weak promoter may cause activation of the plant defense system short of hypersensitive cell death. Thus, there is an activation of the plant defense system at levels sufficient to protect from pathogen invasion. In this state, there is at least a partial activation of the plant defense system wherein the plant produces increased levels of antipathogenic factors such as PR proteins, *i.e.,* PR-1, cattiness, a-glucanases, etc.; secondary metabolites; phytoalexins; reactive oxygen species; and the like.

Alternatively, the heterologous nucleotide sequence operably linked to the constitutive promoter disclosed herein may be an antisense sequence for a targeted gene. By "antisense DNA nucleotide sequence" is intended a sequence that is in inverse orientation to the 5' to 3' normal orientation of that nucleotide sequence. When delivered into a plant cell, expression of the antisense DNA sequence prevents normal expression of the DNA nucleotide sequence for the targeted gene. The antisense nucleotide sequence encodes an RNA transcript that is complementary to and capable of hybridizing to the endogenous messenger RNA (mRNA) produced by transcription of the DNA nucleotide sequence for the targeted gene. In this case, production of the native protein encoded by the targeted gene is inhibited to achieve a desired phenotypic response. Thus the promoter sequence disclosed herein may be operably linked to antisense DNA sequences to reduce or inhibit expression of a native protein in the plant.

The following examples are offered by way of illustration and not by way of limitation.

### EXPERIMENTAL

The promoter region for the maize gene encoding a metallothionein was isolated from maize plants and cloned. This gene was selected as a source of a constitutive promoter based on the developmental and spatial expression of its gene product. The method for its isolation is described below.

Metallothioneins make up a class of proteins that are constitutively expressed in low copy number in cells throughout a plant. Expression of these proteins in mammalian cells is inducible in response to stress and to elevated concentrations of trace elements and hormones. The novel promoter sequence for a maize gene encoding one of these metallothioneins, met-1, is set forth in SEQ ID NO: 1.

### Example 1: Isolation of Promoter Sequence

The procedure for promoter isolation is described in the User Manual for the GenomeWalker kit sold by Clontech Laboratories, Inc., Palo Alto, California. Genomic DNA from maize line A63 was extracted by grinding 10-day old seedling leaves in liquid nitrogen, and the DNA prepared as described by Chen and Dellaporta (1994) in *The Maize Handbook,* ed. Freeling and Walbot (Springer-Verlag, Berlin). RNase A was added to 10 µg/ml and then incubated at 37°C for 1 hr. The DNA was then extracted once with phenol-chloroform, then chloroform, then ethanol precipitated and resuspended in TE (10 mM Tris pH 8.0, 1 mM EDTA). The DNA was then used exactly as described in the GenomeWalker User Manual (Clontech PT3042-1 version PR68687). Briefly, the DNA was digested separately with restriction enzymes Dral, EcoRV, PvuII, ScaI ,and StuI, all blunt end cutters. The GenomeWalker adapters were then ligated onto the ends of the restricted DNA. The resulting DNA is referred to as DL1-DL5, respectively.

For isolation of the specific promoter region, two nonoverlapping gene-specific primers (27 bp in length) were designed from the sequences of abundant ESTs in the Pioneer/HGS database. The primers were designed to amplify the region upstream of the coding sequence, *i.e.,* the 5' untranslated region and promoter region of the gene. The sequences of the primers are given below. The first round of PCR was performed on each DNA sample (DL1-5) with Clontech primer AP1 (sequence 5'-gtaatacgactcactatagggc-3'; SEQ ID NO: 2) and the gene-specific primer (gsp) 1 with the following sequence:
metallothionein gsp1 sequence: 5'-gtacttcttgccgcacttgcagcttga-3' (SEQ ID NO: 3).

PCR was performed in a model PTC-100 thermal cycler with HotBonnet from MJ Research (Watertown, Massachusetts) using reagents supplied with the GenomeWalker kit. The following cycle parameters were used: 7 cycles of 94°C for 2 sec, then 72°C for 3 min, followed by 32 cycles of 94°C for 2 sec, and 67°C for 3 min. Finally, the samples were held at 67°C for 4 min, then at 4°C until further analysis. As described in the User Manual, the DNA from the first round of PCR was then diluted and used as a template in a second round of PCR using the Clontech AP2 primer (sequence 5'-actatagggcacgcgtggt-3'; SEQ ID NO: 4) and gene-specific primer (gsp)2 with the following sequence:
metallothionein gsp2 sequence: 5'agccgcagcttgatccgcagttgcaag-3' (SEQ ID NO: 5).

The cycle parameters for the second round were: 5 cycles of 94°C for 2 sec, then 72°C for 3 min, followed by 20 cycles of 94°C for 2 sec, and 67°C for 3 min. Finally, the samples were held at 67°C for 4 min, and then held at 4°C.

Approximately 10 µl of each reaction were run on a 0.8% agarose gel, and bands (usually 500bp or larger) were excised, purified with the Sephaglas BandPrep kit (Pharmacia, Piscataway, New Jersey), and cloned into the TA vector pCR2.1 (Invitrogen, San Diego, California). Clones were sequenced for verification, and then the mini-prep DNA was diluted 1:30 in water and 1 µl was amplified with gene-specific primer (gsp)3 (sequences below) and the Clontech AP2 primer with the following cycle parameters: five cycles of 94°C for 2 sec, 46°C for 30 sec, 72°C for min, followed by 20 cycles of 94°C for 2 sec, 67°C for 3 min, then 67°C for 4 min, and held at 4°C.
metallothionein gsp3 sequence: 5'-gaccatggtgtcgtgtggatccccttgtggtgc-3' (SEQ ID NO: 6).

Ten µl of the resulting amplified DNA was run out on a 0.8% agarose gel, purified with Sephaglas, and cloned into the PCR2.1 TA vector. The final sequence was determined on the resulting plasmids.

### Example 2: Transient Gene Expression Data Using Promoter Sequence

A transient gene expression assay was used to test the cloned DNA for promoter activity. The promoter was recloned into plasmid PHP3953 (Figure 1) digested with BglII and SphI or with BglII and PvuII to remove the ubiquitin promoter. The new promoter fragment was inserted in place of the ubiquitin promoter such that the ubiquitin 5' untranslated region and intron were now between the test promoter and the GUS reporter gene.

Experiments were performed with immature embryos, essentially the scutellar surface. Immature GS3 maize embryos were isolated from ears 9-11 days after pollination using a scalpel. Prior to embryo isolation, pollinated ears were surface-sterilized with a microdetergent and 25% commercial bleach mixture, then washed with 3 exchanges of sterile H₂O. Isolated immature embryos (1.4-1.7 mm) were placed on bombardment medium and aligned in a target grid in preparation for bombardment.

Immature embryos were then transformed by the tungsten particle biolistic method (Tomes *et al.* (1995) in *Plant Cell, Tissue, and Organ Culture: Fundamental Methods,* ed. Gamborg and Phillips (Springer-Verlag, Berlin); Koziel *et al.* (1993) *Bio*/*Technology 11*:194-200) using a high pressure particle delivery system (Biolistic Particle Delivery System Model PDS-100 by DuPont). Plasmid DNA comprising the promoter sequence of the invention operably linked to the GUS gene was bombarded into the maize immature embryos. Following culture for 40 hours, bombarded embryos were stained with X-Gluc staining solution (McCabe *et al.* (1988) *Bio*/*Technology* 6(87):923-926) for 12 h at 37°C in the dark. GUS activity was then measured, using the ubiquitin promoter as a control, by counting blue spots after staining for GUS activity as described elsewhere (see Jefferson (1987) *Plant Mol. Biol. Rep.* 5:387-405). Transient expression data are shown in Table 1.

**Table 1: Promoter activity as measured by transient expression of GUS (see Christensen and Quail (1989) Plant Mol. Biol. 12:619-632 for details of ubiquitin promoter activity).**

| Gene | #Blue spots | Average |
|---|---|---|
| Ubiquitin (control) | 395, 155, 307, 416 | 318 |
| met-1 (methallothionein) | 163, 111, 27 | 100 |

Promoter activity of the promoter sequence for the metallothionein gene was considerably lower than that of the ubiquitin promoter, indicating this is a weak constitutive promoter.

### Example 3: Transformation and Regeneration of Transgenic Plants

Immature GS3 maize embryos were bombarded with a plasmid containing the promoter sequence operably linked to the GUS gene plus a plasmid containing the selectable marker gene PAT (Wohlleben *et al.* (1988) *Gene* 70:25-37) that confers resistance to the herbicide Bialaphos. Transformation was performed as described in Example 1. See Appendix for examples of bombardment (560Y), selection (560R), hormone-containing regeneration (288J), and hormone-free (272V) media.

One day after bombardment, the embryos were transferred from bombardment medium to selection medium containing 3 mg/liter Bialaphos and subcultured on selection medium every 2 weeks. After approximately 10 weeks of selection, selection-resistant callus clones were transferred to hormone-containing medium to initiate plant regeneration. Following somatic embryo maturation (2-4 weeks), well-developed somatic embryos were transferred to medium for germination and transferred to the lighted culture room. Approximately 7-10 days later, developing plantlets were transferred to hormone-free medium in tubes for 7-10 days until plantlets were well established. Plants were then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity.

Promoter activity in transformed plant tissues was assessed by measuring expression of GUS. Plant tissue samples were placed into the wells of 12-well cell culture clusters and stained with X-Gluc staining solution for 12 h at 37°C in the dark. Samples were then scored for GUS staining. GUS staining score ranged from 0 (negative) - 6 (highest). These staining scores served as a measure of level of GUS expression. Results are shown in Table 2.

Four transformation events have been evaluated for the met-1 promoter. Overall, three events scored strong GUS expression and one event scored median expression. GUS expression was found in all plant tissues, including leaf, root, husk, silk, and tassel, with these tissues mostly having staining scores of 4-5.

The ubiquitin promoter was used as a control. Five transformation events have been evaluated. Overall, two events scored strong GUS expression and three scored weak expression. GUS expression was found in all plant tissues, including leaf, root, husk, silk, and tassel. With those transformation events exhibiting strong expression, GUS was expressed in all different tissues, with tissues mostly having staining scores of 4-5. With those transformation events exhibiting weak expression, GUS was expressed in leaf, root, and tassel, with these tissues mostly having staining scores of 1-3. There was very little GUS expression in husk and silk tissues in those transformation events that exhibited weak expression.

**Table 2. Gus expression as driven by met-1 promoter of the invention.**

| **Promoters** | **Event** | **GUS expression** | **GUS PCR** |
|---|---|---|---|
| met-1 | TC7413 | strong | positive |
| | TC7414 | weak | positive |
| | TC7415 | strong | positive |
| | TC7416 | strong | positive |
| ubi | TC7390 | strong | positive |
| | TC7386 | strong | positive |
| | TC7387 | weak | positive |
| | TC7388 | weak | positive |
| | TC7389 | weak | positive |

### APPENDIX

**272 V**

| Ingredient | Amount | Unit |
|---|---|---|
| D-I H₂O | 950.000 | ml |
| MS Salts (GIBCO 11117-074) | 4.300 | g |
| Myo-Inositol | 0.100 | g |
| MS Vitamins Stock Solution ## | 5.000 | ml |
| Sucrose | 40.000 | g |
| Bacto-Agar @ | 6.000 | g |

| | | |
|---|---|---|
| Directions: | | |
| @ = Add after bringing up to volume Dissolve ingredients in polished D-I H₂O in sequence Adjust to pH 5.6 Bring up to volume with polished D-I H₂O after adjusting pH Sterilize and cool to 60°C. | | |
| ## = Dissolve 0.100 g of Nicotinic Acid; 0.020 g of Thiamine.HCL; 0.100 g of Pyridoxine.HCL; and 0.400 g of Glycine in 875.00 ml of polished D-I H₂O in sequence. Bring up to volume with polished D-I H₂O. Make in 400 ml portions. Thiamine.HCL & Pyridoxine.HCL are in Dark Desiccator. Store for one month, unless contamination or precipitation occurs, then make fresh stock. Total Volume (L) = 1.00 | | |

**288 J**

| Ingredient | Amount | Unit |
|---|---|---|
| D-I H₂O | 950.000 | ml |
| MS Salts | 4.300 | g |
| Myo-Inositol | 0.100 | g |
| MS Vitamins Stock Solution ## | 5.000 | ml |
| Zeatin .5mg/ml | 1.000 | ml |
| Sucrose | 60.000 | g |
| Gelrite @ | 3.000 | g |
| Indoleacetic Acid 0.5 mg/ml # | 2.000 | ml |
| .1mM Abscisic Acid | 1.000 | ml |
| Bialaphos 1mg/ml # | 3.000 | ml |

| | | |
|---|---|---|
| Directions: | | |
| @ = Add after bringing up to volume Dissolve ingredients in polished D-1 H₂O in sequence Adjust to pH 5.6 Bring up to volume with polished D-I H₂O after adjusting pH Sterilize and cool to 60°C. Add 3.5g/L of Gelrite for cell biology. | | |
| ## = Dissolve 0.100 g of Nicotinic Acid; 0.020 g of Thiamine.HCL; 0.100 g of Pyridoxine.HCL; and 0.400 g of Glycine in 875.00 ml of polished D-1 H₂O in sequence. Bring up to volume with polished D-1 H₂O. Make in 400 ml portions. Thiamine.HCL & Pyridoxine.HCL are in Dark Desiccator. Store for one month, unless contamination or precipitation occurs, then make fresh stock. Total Volume (L) = 1.00 | | |

**560 R**

| Ingredient | Amount | Unit |
|---|---|---|
| D-I Water, Filtered | 950.000 | ml |
| CHU (N6) Basal Salts (SIGMA C-1416) | 4.000 | g |
| Eriksson's Vitamin Mix (1000X SIGMA-1511 | 1.000 | ml |
| Thiamine.HCL 0.4mg/ml | 1.250 | ml |
| Sucrose | 30.000 | g |
| 2, 4-D 0.5mg/ml | 4.000 | ml |
| Gelrite @ | 3.000 | g |
| Silver Nitrate 2mg/ml # | 0.425 | ml |
| Bialaphos 1 mg/ml # | 3.000 | ml |

| | | |
|---|---|---|
| Directions: | | |
| @ = Add after bringing up to volume | | |
| # = Add after sterilizing and cooling to temp. Dissolve ingredients in D-I H₂O in sequence Adjust to pH 5.8 with KOH Bring up to volume with D-I H₂O Sterilize and cool to room temp. Total Volume (L) = 1.00 | | |

**560 Y**

| Ingredient | Amount | Unit |
|---|---|---|
| D-I Water, Filtered | 950.000 | ml |
| CHU (N6) Basal Salts (SIGMA C-1416) | 4.000 | g |
| Eriksson's Vitamin Mix (1000X SIGMA-1511 | 1.000 | ml |
| Thiamine.HCL 0.4mg/ml | 1.250 | ml |
| Sucrose | 120.000 | g |
| 2,4-D 0.5mg/ml | 2.000 | ml |
| L-Proline | 2.880 | g |
| Gelrite @ | 2.000 | g |
| Silver Nitrate 2mg/ml # | 4.250 | ml |

| | | |
|---|---|---|
| Directions: | | |
| @ = Add after bringing up to volume | | |
| # = Add after sterilizing and cooling to temp. Dissolve ingredients in D-I H₂O in sequence Adjust to pH 5.8 with KOH Bring up to volume with D-I H₂O Sterilize and cool to room temp. | | |
| ** Autoclave less time because of increased sucrose Total Volume (L) = 1.00 | | |

All publications and patent applications mentioned in the specification are indicative of the level of those skilled in the art to which this invention pertains.

Although the foregoing invention has been described in some detail by way of illustration and example for purposes of clarity of understanding, it will be obvious that certain changes and modifications may be practiced within the scope of the appended claims.

## Claims

1. An isolated nucleic acid molecule having a nucleotide sequence for a promoter that is capable of initiating transcription in a plant cell, wherein said nucleotide sequence is selected from:
(a) a nucleotide sequence comprising the sequence set forth in SEQ ID NO: 1;
(b) a nucleotide sequence deposited as ATCC Accession No. 207120;
(c) a nucleotide sequence comprising at least 40 contiguous nucleotides of the sequence set forth in SEQ ID NO: 1;
(d) a nucleotide sequence that hybridizes under stringent conditions to a sequence of a), b) or c); and
(e) a nucleotide sequence that has at least 70% sequence identity to a sequence set forth in a) or b).

2. A DNA construct comprising a nucleotide sequence of claim 1 operably linked to a heterologous nucleotide sequence of interest.

3. A vector comprising the DNA construct of claim 2.

4. A host cell having stably incorporated in its genome the DNA construct of claim 2.

5. A method of constitutively expressing a heterologous nucleotide sequence in a plant, said method comprising transforming a plant cell with a DNA construct as defined in claim 2.

6. A method of claim 5, wherein said plant is a monocot or a dicot.

7. The method of claim 6, wherein said monocot is maize.

8. A plant cell stably transformed with a DNA construct as defined in claim 2.

9. A plant stably transformed with a DNA construct as defined in claim 2.

10. A plant cell of claim 8 wherein said plant cell is from a monocot or a dicot.

11. A plant of claim 9 wherein said plant is a monocot or a dicot.

12. A plant cell of claim 10 or plant of claim 11, wherein said monocot is maize.

13. Seed of the plant of claim 9, 11 or 12 comprising a DNA construct of claim 2.

## Patentansprüche

1. Isoliertes Nukleinsäuremolekül mit einer Nukleotidsequenz für einen Promotor, der fähig ist, die Transkription in einer Pflanzenzelle zu initiieren, wobei die Nukleotidsequenz ausgewählt ist aus:
a) einer Nukleotidsequenz, die die in SEQ ID NO: 1 dargestellte Sequenz umfasst;
b) einer Nukleotidsequenz, die mit der Eingangsnummer ATCC No. 207120 hinterlegt ist;
c) einer Nukleotidsequenz, die mindestens 40 aufeinanderfolgende Nukleotide der Sequenz umfasst, die in SEQ ID NO: 1 dargestellt ist;
d) einer Nukleotidsequenz, die unter stringenten Bedingungen mit einer Sequenz von a), b) oder c) hybridisiert; und
e) einer Nukleotidsequenz, die mindestens 70% Sequenzidentität zu einer Sequenz aufweist, die in a) oder b) angeführt ist.

2. DNA-Konstrukt, das eine Nukleotidsequenz nach Anspruch 1, funktionell verknüpft mit einer interessierenden heterologen Nukleotidsequenz, umfasst.

3. Vektor, der das DNA-Konstrukt nach Anspruch 2 umfasst.

4. Wirtszelle, in deren Genom das DNA-Konstrukt nach Anspruch 2 stabil eingebaut ist.

5. Verfahren zur konstitutiven Expression einer heterologen Nukleotidsequenz in einer Pflanze, wobei das Verfahren das Transformieren einer Pflanzenzelle mit einem DNA-Konstrukt, wie es in Anspruch 2 definiert ist, umfasst.

6. Verfahren nach Anspruch 5, wobei die Pflanze eine monokotyledone oder eine dikotyledone Pflanze ist.

7. Verfahren nach Anspruch 6, wobei die monokotyledone Pflanze Mais ist.

8. Pflanzenzelle, die stabil mit einem DNA-Konstrukt, wie es in Anspruch 2 definiert ist, transformiert ist.

9. Pflanze, die stabil mit einem DNA-Konstrukt, wie es in Anspruch 2 definiert ist, transformiert ist.

10. Pflanzenzelle nach Anspruch 8, wobei die Pflanzenzelle von einer monokotyledonen oder dikotyledonen Pflanze stammt.

11. Pflanze nach Anspruch 9, wobei die Pflanze eine monokotyledone oder dikotyledone Pflanze ist.

12. Pflanzenzelle nach Anspruch 10 oder Pflanze nach Anspruch 11, wobei die monokotyledone Pflanze Mais ist.

13. Samen der Pflanze nach Anspruch 9, 11 oder 12, umfassend ein DNA-Konstrukt nach Anspruch 2.

## Revendications

1. Molécule d'acide nucléique isolée ayant une séquence nucléotidique pour un promoteur qui est capable d'initier la transcription dans une cellule végétale, dans laquelle ladite séquence nucléotidique est choisie parmi :
a) une séquence nucléotidique comprenant la séquence présentée en SEQ ID NO : 1 ;
b) une séquence nucléotidique déposée sous le n° d'accès ATCC 207120 ;
c) une séquence nucléotidique comprenant au moins 40 nucléotides contigus de la séquence présentée en SEQ ID NO :1 ;
d) une séquence nucléotidique qui s'hybride dans des conditions stringentes avec une séquence de a), b) ou c) ; et
e) une séquence nucléotidique qui a au moins une identité de séquence de 70 % avec une séquence présentée en a) ou b).

2. Construction d'ADN comprenant une séquence nucléotidique de la revendication 1 liée de manière opérable avec une séquence nucléotidique hétérologue d'intérêt.

3. Vecteur comprenant la construction d'ADN de la revendication 2.

4. Cellule hôte ayant, incorporée de manière stable dans son génome, la construction d'ADN de la revendication 2.

5. Méthode pour exprimer de manière constitutive une séquence nucléotidique hétérologue dans une plante, ladite méthode comprenant le fait de transformer une cellule végétale avec une construction d'ADN telle que définie à la revendication 2.

6. Méthode de la revendication 5, dans laquelle ladite plante est une monocotylédone ou une dicotylédone.

7. Méthode de la revendication 6, dans laquelle ladite monocotylédone est du maïs.

8. Cellule végétale transformée de manière stable avec une construction d'ADN telle que définie à la revendication 2.

9. Plante transformée de manière stable avec une construction d'ADN telle que définie à la revendication 2.

10. Cellule végétale de la revendication 8 dans laquelle ladite cellule végétale provient d'une monocotylédone ou d'une dicotylédone.

11. Plante végétale de la revendication 9 dans laquelle ladite plante est une monocotylédone ou une dicotylédone.

12. Cellule végétale de la revendication 10 ou plante de la revendication 11, dans laquelle ladite monocotylédone est du maïs.

13. Graine de la plante de la revendication 9, 11 ou 12 comprenant une construction d'ADN de la revendication 2.


### SEQUENCE LISTING

<110> Pioneer Hi-Bred International, Inc.
<120> Maize Met-1 Promoter
<130> N80441A
<150> 60/076,075
   <151> 1998-02-26
<160> 6
<170> PatentIn Ver. 2.0
<210> 1
   <211> 1300
   <212> DNA
   <213> Zea mays
<400> 1
   gaatataggc aacaccccac actggtaggt aaagctccgg gattgtgact gagatgggga 60
   tgatgacgtt gtagagaata caggcagccg tcggcgctac aactctcctc acaaagaaga 120
   agctatagag catatagaac ttcttgacga gtgatatatc ctagcggaac aacaagtggg 180
   gctaattttt agattttccc ctgcgaaagg catgatttgc cagaatggga acctgggcag 240
   atatatgctc accttggcaa acaaaacatc ctttgccatc ttacggaata agtttgcacc 300
   acctagacca ccggaattgc tgccgacagt aggccttgta agtggacggc agttcactct 360
   tgacctgtaa atataacatg tttgcatagt caaatgatca catcaaatat atttaatttt 420
   ggcaacactc ttatatgtca aaatctactc actctaacgt ctccaacata tacgaatttc 480
   cagcccttta gtgttgctcg aaccgccaag tccatgcctt caactgtagt tcggtccttc 540
   caacctcctg catctcttat ggctcctgta cgccacactc cagcagttcc tagaatagac 600
   ataccatcag gaagaattta tggtcttgaa attgacattc tggggtggtt cccctctttt 660
   ggggaatttg tttgtactct ttagaggttt caaaagatgt gtaccgttga aactgaagaa 720
   ggcaaaggtc gctgatcctg cttcttgttc aactttgaag tggtagtcgt aaaacatctt 780
   ttgtaccctt gtcagcaggc ttgtcgtgtc attcactgca aaattctgtc aattaagaat 840
   tggaccgatc agagtgaata aaaatactat atcgctcgag aagcttatgc actagcagtt 900
   ctctttttca tgttatatca tctttgaaca gttctatgcc atctttctgg catcatcatt 960
   aaagaatgtg aaaatatatt ttattgtcat cattatgaaa cgtgatgcct attttataag 1020
   cccaaaacag tgaggttgtc tgttccaagt tccttgtgct atttgtaaga actatctgtg 1080
   cacttctcac aagaaaaaca tctgaaaatg gagaaagtat tccattgtgg tggtatactg 1140
   aaatgtggga gagcatatct gacaaatttg acatgaggtt taagaaatat ttcccatagt 1200
   atttttctat tccataaatt aatcatgtgc atttgttata gacgggtagc tggaaaagtg 1260
   tgctcaagaa ctagcaggga atggaccagc caggccgtcg 1300
<210> 2
   <211> 22
   <212> DNA
   <213> Artificial Sequence
<223> Description of Artificial Sequence: PCR Primer
<400> 2
   gtaatacgac tcactatagg gc 22
<210> 3
   <211> 27
   <212> DNA
   <213> Artificial Sequence
<223> Description of Artificial Sequence: Maize gene-specific PCR primer
<400> 3
   gtacttcttg ccgcacttgc agcttga 27
<210> 4
   <211> 19
   <212> DNA
   <213> Artificial Sequence
<223> Description of Artificial Sequence: Maize gene-specific PCR primer
<400> 4
   actatagggc acgcgtggt 19
<210> 5
   <211> 27
   <212> DNA
   <213> Artificial Sequence
<223> Description of Artificial Sequence: Maize gene-specific PCR primer
<400> 5
   agccgcagct tgatccgcag ttgcaag 27
<210> 6
   <211> 33
   <212> DNA
   <213> Artificial Sequence
<223> Description of Artificial Sequence: Maize gene-specific PCR primer
<400> 6
   gaccatggtg tcgtgtggat ccccttgtgg tgc 33

### SEQUENCE LISTING

<110> Pioneer Hi-Bred International, Inc.
<120> Maize Met-1 Promoter
<130> N80441A
<150> 60/076,075
   <151> 1998-02-26
<160> 6
<170> PatentIn Ver. 2.0
<210> 1
   <211> 1300
   <212> DNA
   <213> Zea mays
<400> 1
   gaatataggc aacaccccac actggtaggt aaagctccgg gattgtgact gagatgggga 60
   tgatgacgtt gtagagaata caggcagccg tcggcgctac aactctcctc acaaagaaga 120
   agctatagag catatagaac ttcttgacga gtgatatatc ctagcggaac aacaagtggg 180
   gctaattttt agattttccc ctgcgaaagg catgatttgc cagaatggga acctgggcag 240
   atatatgctc accttggcaa acaaaacatc ctttgccatc ttacggaata agtttgcacc 300
   acctagacca ccggaattgc tgccgacagt aggccttgta agtggacggc agttcactct 360
   tgacctgtaa atataacatg tttgcatagt caaatgatca catcaaatat atttaatttt 420
   ggcaacactc ttatatgtca aaatctactc actctaacgt ctccaacata tacgaatttc 480
   cagcccttta gtgttgctcg aaccgccaag tccatgcctt caactgtagt tcggtccttc 540
   caacctcctg catctcttat ggctcctgta cgccacactc cagcagttcc tagaatagac 600
   ataccatcag gaagaattta tggtcttgaa attgacattc tggggtggtt cccctctttt 660
   ggggaatttg tttgtactct ttagaggttt caaaagatgt gtaccgttga aactgaagaa 720
   ggcaaaggtc gctgatcctg cttcttgttc aactttgaag tggtagtcgt aaaacatctt 780
   ttgtaccctt gtcagcaggc ttgtcgtgtc attcactgca aaattctgtc aattaagaat 840
   tggaccgatc agagtgaata aaaatactat atcgctcgag aagcttatgc actagcagtt 900
   ctctttttca tgttatatca tctttgaaca gttctatgcc atctttctgg catcatcatt 960
   aaagaatgtg aaaatatatt ttattgtcat cattatgaaa cgtgatgcct attttataag 1020
   cccaaaacag tgaggttgtc tgttccaagt tccttgtgct atttgtaaga actatctgtg 1080
   cacttctcac aagaaaaaca tctgaaaatg gagaaagtat tccattgtgg tggtatactg 1140
   aaatgtggga gagcatatct gacaaatttg acatgaggtt taagaaatat ttcccatagt 1200
   atttttctat tccataaatt aatcatgtgc atttgttata gacgggtagc tggaaaagtg 1260
   tgctcaagaa ctagcaggga atggaccagc caggccgtcg                       1300
<210> 2
   <211> 22
   <212> DNA
   <213> Artificial Sequence
<223> Description of Artificial Sequence: PCR Primer
<400> 2
   gtaatacgac tcactatagg gc 22
<210> 3
   <211> 27
   <212> DNA
   <213> Artificial Sequence
<223> Description of Artificial Sequence: Maize gene-specific PCR primer
<400> 3
   gtacttcttg ccgcacttgc agcttga 27
<210> 4
   <211> 19
   <212> DNA
   <213> Artificial Sequence
<223> Description of Artificial Sequence: Maize gene-specific PCR primer
<400> 4
   actatagggc acgcgtggt 19
<210> 5
   <211> 27
   <212> DNA
   <213> Artificial Sequence
<223> Description of Artificial Sequence: Maize gene-specific PCR primer
<400> 5
   agccgcagct tgatccgcag ttgcaag 27
<210> 6
   <211> 33
   <212> DNA
   <213> Artificial Sequence
<223> Description of Artificial Sequence: Maize gene-specific PCR primer
<400> 6
   gaccatggtg tcgtgtggat ccccttgtgg tgc 33

### SEQUENCE LISTING

<110> Pioneer Hi-Bred International, Inc.
<120> Maize Met-1 Promoter
<130> N80441A
<150> 60/076,075
   <151> 1998-02-26
<160> 6
<170> PatentIn Ver. 2.0
<210> 1
   <211> 1300
   <212> DNA
   <213> Zea mays
<400> 1
   gaatataggc aacaccccac actggtaggt aaagctccgg gattgtgact gagatgggga 60
   tgatgacgtt gtagagaata caggcagccg tcggcgctac aactctcctc acaaagaaga 120
   agctatagag catatagaac ttcttgacga gtgatatatc ctagcggaac aacaagtggg 180
   gctaattttt agattttccc ctgcgaaagg catgatttgc cagaatggga acctgggcag 240
   atatatgctc accttggcaa acaaaacatc ctttgccatc ttacggaata agtttgcacc 300
   acctagacca ccggaattgc tgccgacagt aggccttgta agtggacggc agttcactct 360
   tgacctgtaa atataacatg tttgcatagt caaatgatca catcaaatat atttaatttt 420
   ggcaacactc ttatatgtca aaatctactc actctaacgt ctccaacata tacgaatttc 480
   cagcccttta gtgttgctcg aaccgccaag tccatgcctt caactgtagt tcggtccttc 540
   caacctcctg catctcttat ggctcctgta cgccacactc cagcagttcc tagaatagac 600
   ataccatcag gaagaattta tggtcttgaa attgacattc tggggtggtt cccctctttt 660
   ggggaatttg tttgtactct ttagaggttt caaaagatgt gtaccgttga aactgaagaa 720
   ggcaaaggtc gctgatcctg cttcttgttc aactttgaag tggtagtcgt aaaacatctt 780
   ttgtaccctt gtcagcaggc ttgtcgtgtc attcactgca aaattctgtc aattaagaat 840
   tggaccgatc agagtgaata aaaatactat atcgctcgag aagcttatgc actagcagtt 900
   ctctttttca tgttatatca tctttgaaca gttctatgcc atctttctgg catcatcatt 960
   aaagaatgtg aaaatatatt ttattgtcat cattatgaaa cgtgatgcct attttataag 1020
   cccaaaacag tgaggttgtc tgttccaagt tccttgtgct atttgtaaga actatctgtg 1080
   cacttctcac aagaaaaaca tctgaaaatg gagaaagtat tccattgtgg tggtatactg 1140
   aaatgtggga gagcatatct gacaaatttg acatgaggtt taagaaatat ttcccatagt 1200
   atttttctat tccataaatt aatcatgtgc atttgttata gacgggtagc tggaaaagtg 1260
   tgctcaagaa ctagcaggga atggaccagc caggccgtcg                       1300
<210> 2
   <211> 22
   <212> DNA
   <213> Artificial Sequence
<223> Description of Artificial Sequence: PCR Primer
<400> 2.
   gtaatacgac tcactatagg gc 22
<210> 3
   <211> 27
   <212> DNA
   <213> Artificial Sequence
<223> Description of Artificial Sequence: Maize gene-specific PCR primer
<400> 3
   gtacttcttg ccgcacttgc agcttga 27
<210> 4
   <211> 19
   <212> DNA
   <213> Artificial Sequence
<223> Description of Artificial Sequence: Maize gene-specific PCR primer
<400> 4
   actatagggc acgcgtggt 19
<210> 5
   <211> 27
   <212> DNA
   <213> Artificial Sequence
<223> Description of Artificial Sequence: Maize gene-specific PCR primer
<400> 5
   agccgcagct tgatccgcag ttgcaag 27
<210> 6
   <211> 33
   <212> DNA
   <213> Artificial Sequence
<223> Description of Artificial Sequence: Maize gene-specific PCR primer
<400> 6
   gaccatggtg tcgtgtggat ccccttgtgg tgc 33

### SEQUENCE LISTING

<110> Pioneer Hi-Bred International, Inc
<120> Maize Met-1 Promoter
<130> N80441A
   <150> 60/076,075
   <151> 1998-02-26
<160> 34
<170> PatentIn Ver. 2.0
<210> 1
   <211> 1392
   <212> DNA
   <213> Zea mays
<400> 1
   cgacggcccg ggctggtatc gataaatgtt tccacataga ttttgcatat cataatgatg 60
   tttgtcgttc cgtatctatg tttcatacaa aatttttacg catatcgcaa cacatgggca 120
   catacctagt gactgtataa ctctgcatgt atgagtgtat gactatatga tgtagtaact 180
   aataagaagg gtagacattt gagtgattct tttattcctg gacttgtaag acttgacatt 240
   tctgccttga gtgcgataca tcatatggac aggggttatg catacactgc ttgtttgttg 300
   tttatgttct aagagcatct ccaacaacgt gacatatgaa aatgccctac aatttaaaaa 360
   tggttatatt ttataaaatt tagggcataa ataaaacatc ccgctccaac attaaagcct 420
   taaatctatt atagggaagc ccactatgat atagtatatt tgaggcactt tagagggtgc 480
   cctataattt tttgaccatt tttttatgaa atgagacact attggagtat tttttttccg 540
   tagagcacca tatttcaatt tgagacacca atttaaggca ttgttggaga tgttctaaat 600
   gttggtttat tttgtctgta tcgttgtggt tttgatagtg gtgcctttgc aatgtacatc 660
   ttacattgac aataataata ggtaaaactc tacaaatttt ttatctaatg gactcttgta 720
   tgaaacattg tacttgcaca catctgatgt aaacactgca tacttttaac agtgacaaga 780
   ttctgtttca ttttagggct agtttgggaa ccaaatttta ttagggtttt tattttctaa 840
   gaaaaagtaa tttattttac cttgagaaaa tataaattac ttgagaaaat agagttccaa 900
   actagctctt atctttgtcg aatcctcctc tattcaaatg tgacatttct ggcacgtgac 960
   aactggtgat gttgtagact gtgttaagta atacgtgtca ttattactaa atgccatttt 1020
   agtaaatgtt gagtatgtac tctactacag taagtattat tggtgtattt acactagaca 1080
   gttggcggcc tggcgggtaa agttatcctg tagaaagttg ggccaggcca aaaccaaccg 1140
   ccaaaggaaa ggccttccgg cccgcccacc tttgcgcgcc gaaggtcagt tccttcagtc 1200
   tcctcccgct tcagactctg accacgtcga caatccgggc cgaaacacat ctgcaccgtc 1260
   cacttgcgac agattgaaca caccacttct atccacgtca gcgatccgtg gcactagccc 1320
   ttccaccaat cagcccaagt tgcccctttc ctttaaattc gccgcaccca ttgctcttct 1380
   cacggccata ga                                                   1392
<210> 2
   <211> 1300
   <212> DNA
   <213> Zea mays
<400> 2
   gaatataggc aacaccccac actggtaggt aaagctccgg gattgtgact gagatgggga 60
   tgatgacgtt gtagagaata caggcagccg tcggcgctac aactctcctc acaaagaaga 120
   agctatagag catatagaac ttcttgacga gtgatatatc ctagcggaac aacaagtggg 180
   gctaattttt agattttccc ctgcgaaagg catgatttgc cagaatggga acctgggcag 240
   atatatgctc accttggcaa acaaaacatc ctttgccatc ttacggaata agtttgcacc 300
   acctagacca ccggaattgc tgccgacagt aggccttgta agtggacggc agttcactct 360
   tgacctgtaa atataacatg tttgcatagt caaatgatca catcaaatat atttaatttt 420
   ggcaacactc ttatatgtca aaatctactc actctaacgt ctccaacata tacgaatttc 480
   cagcccttta gtgttgctcg aaccgccaag tccatgcctt caactgtagt tcggtccttc 540
   caacctcctg catctcttat ggctcctgta cgccacactc cagcagttcc tagaatagac 600
   ataccatcag gaagaattta tggtcttgaa attgacattc tggggtggtt cccctctttt 660
   ggggaatttg tttgtactct ttagaggttt caaaagatgt gtaccgttga aactgaagaa 720
   ggcaaaggtc gctgatcctg cttcttgttc aactttgaag tggtagtcgt aaaacatctt 780
   ttgtaccctt gtcagcaggc ttgtcgtgtc attcactgca aaattctgtc aattaagaat 840
   tggaccgatc agagtgaata aaaatactat atcgctcgag aagcttatgc actagcagtt 900
   ctctttttca tgttatatca tctttgaaca gttctatgcc atctttctgg catcatcatt 960
   aaagaatgtg aaaatatatt ttattgtcat cattatgaaa cgtgatgcct attttataag 1020
   cccaaaacag tgaggttgtc tgttccaagt tccttgtgct atttgtaaga actatctgtg 1080
   cacttctcac aagaaaaaca tctgaaaatg gagaaagtat tccattgtgg tggtatactg 1140
   aaatgtggga gagcatatct gacaaatttg acatgaggtt taagaaatat ttcccatagt 1200
   atttttctat tccataaatt aatcatgtgc atttgttata gacgggtagc tggaaaagtg 1260
   tgctcaagaa ctagcaggga atggaccagc caggccgtcg                       1300
<210> 3
   <211> 166
   <212> DNA
   <213> Zea mays
<400> 3
   cgacggcccg ggctggtcca acctccaagc ctacaaccag atcagccggt ctggtactgt 60
   gcttctgtgg cacgaaaaaa ccgaccgttg cacggacgag aaacccgaac cgtcgaaaca 120
   atcgtaatcc ccaggggctc aaacgcaaaa caccgtccgc tcccct                166
<210> 4
   <211> 1023
   <212> DNA
   <213> Zea mays
<400> 4
   tgttaacaag acgaatcaat gattagaaac actagtaaat taatactacc agtaacgaca 60
   caatttatcg gatgggatca gaaactagat ctaacgttgt cgccggagtt atgagcttga 120
   aaaaagcttg atacacataa gcagtgtaaa aataaagaag caaatattaa acagagcaat 180
   caatgtaata taaaaaataa agcaggcata gaaaataaag ggcaaactga taagcgtgcg 240
   cacatgattt tgagccagta agacgaattc aaaattaggc acaacaactc tataaagcag 300
   atcaggaatt tggtcatatt atgaggttac aactcttact gcgcatgaac tacaaagaca 360
   agtatcggtc aaatagaaac tacagcagct ctacacctca aaaattcgat ctgactttat 420
   taagaccacg taacacgacc gcaagcgcaa ttaagaagtc gtccaactac ggtggcacgg 480
   tgcgtatgta atttatcaaa tccagaagca actatccgta agaatcaggt catccaaaca 540
   cggaagcaaa caaaaccaca aaaactaaga gaatggagac gcagtagcta caattaatcg 600
   caaacagaag gccgaatcac ttcaggcacg gggaggcaac agagcacagc tttgaaccac 660
   tgcctatctc atcgagcacc tcaaggacaa agatcgagcg acagaggatt agctaccttg 720
   caagggacag aggccgaaat ggaggcgcac aaggcagacg gcgagttggg gaggaaggtg 780
   ctggcctcta gggttcggga ctttatataa gggtgtcgga gtggccggac cagcccaata 840
   cttgacttcc gaatggcggc ccagggccca taaacggagc gagacatagt atatgggatg 900
   actgaatgac aatgttattt gctcttaata ttgtatggca gcacttttag gtatatgccc 960
   caatttctct aatactctac tctttcatat tctaatggca tctcatgtta tgttggccta 1020
   aat 1023
<210> 5
   <211> 1022
   <212> DNA
   <213> Zea mays
<400> 5
   gttaacaaga cgaatcaacg attagaaaca ctagtaaata ttaatactac cagtaacgac 60
   acaatttatc agatgggatc agaaactaga tctaacgttg tcaccggagt tatgagcttg 120
   aaaaaagctt gatacacata agcagtgtaa aaataaagaa gcaaatatta aacagagcaa 180
   tcaatgtaat ataaaaaaat aaagcaggca tagaaaataa agagcaaact aataagtgtg 240
   cgcacataat tttgagccag taagacgaat tcaaaattag gcacaacaac tctataaagc 300
   agatcaggaa tttggtcata ttatgaggtt acaactctta ctgcgcatga actacaaaga 360
   caagtatcgg tcaaatagaa actacagcag ctctacacct caaaaattcg atctgacttt 420
   attaagacca cgtagcacga ccgcaagcgc aattaagaag tcgtccaact acggtggcac 480
   ggtgcgtatg taatttatca aatccagaag caactatccg taagaatcag gtcatccaaa 540
   cacggaagca aacaaaacca caaaaaacta agagaatgga gacgcagtag ctacaattaa 600
   tcgcaaacag aagaccgaat cacttcaggc acggggaggc aacagagcac agctttgaac 660
   cactgcctat ctcatcgagc acctcaagga caaagatcga gcgacagagg attagctacc 720
   ttgcaaggga cagaggccga aatggaggcg cagaaggcag acggcgagtt ggggaggaag 780
   gtgcttgcct ctagggttcg ggactttata taagggtgtc ggtgtggccg gaccagccca 840
   atgtgacttc cgatttccga atggcggccc agggccataa acggagcgag acagggtgtg 900
   tttggttcgg tttttttctg accagcttat atgaaaagct ggttgtgggg aaaagctggc 960
   tgttgggaaa aactggtggc taaaatttag gtgtttggtt cgccagacca gcccgggccg 1020
   tc 1022
<210> 6
   <211> 1769
   <212> DNA
   <213> Zea mays
<400> 6
   cgacggcccg ggctggtctg acttcagtgt aattcaaagc cggaacagat aaaagtgtgt 60
   ctttgccgat gatgttaaat agagtaatac ctgattattg ctgtcaagtg gttgtgttag 120
   taattggaat actgttggag tatgtttccc tttatctttc cattttatca tctgtcagtg 180
   ctcgtgactt tatggtctct aaatttctta gatagttcat gtcgtaccat gaaatgtgtg 240
   tctgtaacat tggtcaacca tattgttcat gtcttcaact ctatttcgaa gtcacaaact 300
   gatcacttgg agtttcggac gtgaagcaac gacaggcatc aatgcctact tcatatataa 360
   aactgcttgc ttgttgcatg ctgttacagc acctaagacc taagtacata cttgcttcct 420
   tttcttattt gtatgctggg tcccatggac cccgttttgt tttgggtttc cctacacatt 480
   gtccccggac ttacagatga caaatatgat gtgttctatg agtcacgtcg aaagaaagaa 540
   atgtacttta gcatttcact gtacataagt ccatcgattt cacattatac tcttctacct 600
   aacaaagccc aaaattgagc tacacatctt tttgatatta gtacgttaat gttagtcaat 660
   tgttatcagg cagcgatgtc tgattataga tctcaaagta agactgtcat ttccccttag 720
   ttcagttagt atagttgctt aaattcagca agtctgaata cttcaacaag gcacaataag 780
   ggaggttcag cacgtaagtt agtataggac catgtgtaat ctatttaaca gagaaaatag 840
   gcataaaaac gactagcctg cagtcgtgcg gatatttgaa tataagtgtt cagatgggcg 900
   agcgcccttg tgctttctct attcgctaaa attcttgaaa agcatgtaag cttttcgcaa 960
   ggttcgttag cagtgactac ctgtttgtag tacatgaaca ttagtatttt acttccctca 1020
   gttcaccttt cttgtgcttg caatacaata cattattttt tacggtgaat ttataaacca 1080
   tatttggtaa gttatattta tactagccta atgcactcga ctcttctatt tagattaagt 1140
   ttggtgaata atttatatgt ttgtgtagcg aaagactcat atttatttag tttttttctt 1200
   gtcaaggata ggacataatt tggtgctttg cttgtgtaat tatgtctacg cacttaaaaa 1260
   atggcacttt ccttacgcct tggtacagtg cttcaataat gagtggataa atgttttatg 1320
   aagttgatct tatttttttg agaattcagc attaataatc atgcgccaca ttatccaaag 1380
   cttatggtat tctttatatg attttagtgc taataatgtt gtgccactac agggcggtgc 1440
   cggctctgat ctggtttata aagtagcaaa ttttgaattt gttttgttgg ctctaaattc 1500
   tgtgtgaaca cttattagtc tgctctgttt attctttagt gcttcttata ttatattgtt 1560
   tgctctattg aatgtttcta ggtctgcttt atcccttgct aacatgtacc atgctttttc 1620
   taagctgatt acagtagttg caactccaaa tttagtttct aattccatct aataattttt 1680
   tttgttagtg gctttagtat tgtgtgattt ttacttgcgt tcctaataat ttattcgttt 1740
   gattaacagt ttaagcttca cggatccac                                   1769
<210> 7
   <211> 461
   <212> DNA
   <213> Zea mays
<400> 7
   ggtacagaag tgagagaagg aaaccctagg tatcgagtgt gtatatagga ggagggaagg 60
   tggggcgcct gggccgcata aaatgtggaa aggctctgaa ccgacaaatt tggatgttta 120
   acagacctgt aaatggttct gtactctaaa atatagtata tagagaatac atgcatcttt 180
   ttatctccaa caaaagtctg taaagaatca tctaaagttt agagcacggc atgcatccgc 240
   taaaaatgaa gggcgtacgg ttctactcta tacactgtgc ttatttattg tgtttgctca 300
   ttcacatgca catgaccggt aaaaatcaat atagaatgtg aaatatggta cactattgat 360
   atagaggatg aaatttagag gacattgctg gagatggaga aaatatagag aacaaaattt 420
   tttagagagt gctgtaaagg actgagaata ttcttttang g                     461
<210> 8
   <211> 467
   <212> DNA
   <213> Zea mays
<400> 8
   gtagcgagac ttgcagatcg atggtggctt gaggtgctgt ggacttgtgg aggcgacgaa 60
   ggtatttaaa gcgacggagg gaaacaccgc cgcgagcaag ttctatcttg aatgttgcca 120
   gaaaataaaa aataaatctg atgcttggat gccattggtg cgtccgggat gctggaacac 180
   ttggcgcgac gggagcctca gggggacgat gaggcagatg tgggctgtgg ctgaggctag 240
   gaatctccgt ggagaagggt gattgggcgc cgtaaatact actatatacc tttgttccaa 300
   gatgtctgtt ttaacttctc taactttaac tgagttcata taaaaaatcc aaacatatta 360
   atcaacttan ttataaattt gcctaaattt aaataagctc tacttacgac aaaaccatat 420
   taaacgtttt aaaacgtcca caacccccaa ttttgcaatg caaaagg 467
<210> 9
   <211> 467
   <212> DNA
   <213> Zea mays
<400> 9
   gtagcgagac ttgcagatcg atggtggctt gagatgctgt ggacttgtgg aggcgacgaa 60
   ggtatttaaa gcgacggagg gaaacctaca atacggagta tgagctaatc taggcggcat 120
   ctcccagtca ccttgatggt gccaatgaat aattatttag ttcgttttat tgttatcatt 180
   tatttttgta agactcttcc actatgtaat aatgattatg acatttatct ctatacactt 240
   tgtcattata tgtgatgttc tcctttggcg cacatatgag acgcacccgt ctttatccct 300
   taaatttggg tgtgacactc cgaaagccct atacaatcac tataaaatct gccaagacct 360
   gggatttgat tgttgtcctc agctcgaaag tgacattgta cccagggaat tcggctgccc 420
   atttggctat tctggcagat gccttgagaa ttctaaatan gtctccc 467
<210> 10
   <211> 565
   <212> DNA
   <213> Zea mays
<400> 10
   taacagtacc aacttgcaca tcagaagccc cgcgagcaca gaagtgtata gaacatatgg 60
   atctgctagc aagtattcct taagaagtat ttctgcttta tacttatagg ccttagctgc 120
   catgattggg tcaacaggaa aatccataat ctgatccatc aagtcaacag tcagattgtt 180
   gaacagagtg gttccattcc ccacaatcca taagcaatgc ctacaacaaa ttgttaaaaa 240
   ttaaatagtt ttattatcat gcaccagata aaagattatg aagtaattga agtgaaattt 300
   aaatgcttgg cgcagaactt acttggcccc tgttaacaat atatagtata gatatgtgct 360
   cgtgcgttgc gatggaagta aaaaatttgt atgaaacatt gatacggaac gacaaaaatc 420
   actataatat gcaaaatata cgtggaaaat attatcaata tcacacaaat taattctaaa 480
   aagttaattt agaattttta attacagaca aatatgtcga caactaatca gctaatcctt 540
   tttagcgata ccagcccggg ccgtc 565
<210> 11
   <211> 22
   <212> DNA
   <213> Artificial Sequence
<220>
<223> Description of Artificial Sequence: PCR Primer
<400> 11
   gtaatacgac tcactatagg gc 22
<210> 12
   <211> 27
   <212> DNA
   <213> Artificial Sequence
<220>
   <223> Description of Artificial Sequence: Maize gene-specific PCR primer
<400> 12
   cgcgggctcc tcctccgccg gcttctt 27
<210> 13
   <211> 27
   <212> DNA
   <213> Artificial Sequence
<220>
   <223> Description of Artificial Sequence: Maize gene-specific PCR primer
<400> 13
   gtacttcttg ccgcacttgc agcttga 27
<210> 14
   <211> 27
   <212> DNA
   <213> Artificial Sequence
<220>
   <223> Description of Artificial Sequence: Maize gene-specific PCR primer
<400> 14
   ggtcttgtca ccgggcatct gaccatc 27
<210> 15
   <211> 27
   <212> DNA
   <213> Artificial Sequence
<220>
   <223> Description of Artificial Sequence: Maize gene-specific PCR primer
<400> 15
   gtcctgtggt ggtcgacttg ccagagt 27
<210> 16
   <211> 27
   <212> DNA
   <213> Artificial Sequence
<220>
   <223> Description of Artificial Sequence: Maize gene-specific PCR primer
<400> 16
   ttcttcctcc aggccttctg ctttcca 27
<210> 17
   <211> 27
   <212> DNA
   <213> Artificial Sequence
<220>
   <223> Description of Artificial Sequence: Maize gene-specific PCR primer
<400> 17
   aggagacgac gacggcacgt tcacggc 27
<210> 18
   <211> 27
   <212> DNA
   <213> Artificial Sequence
<220>
   <223> Description of Artificial Sequence: Maize gene-specific PCR primer
<400> 18
   taatcggtgc tgatgaaggg gtcgttc 27
<210> 19
   <211> 19
   <212> DNA
   <213> Artificial Sequence
<220>
   <223> Description of Artificial Sequence: Maize gene-specific PCR primer
<400> 19
   actatagggc acgcgtggt 19
<210> 20
   <211> 27
   <212> DNA
   <213> Artificial Sequence
<220>
   <223> Description of Artificial Sequence: PCR primer
<400> 20
   gggcttcttc tcggccttgg gcgccat 27
<210> 21
   <211> 27
   <212> DNA
   <213> Artificial Sequence
<220>
   <223> Description of Artificial Sequence: Maize gene-specific PCR primer
<400> 21
   agccgcagct tgatccgcag ttgcaag 27
<210> 22
   <211> 27
   <212> DNA
   <213> Artificial Sequence
<220>
   <223> Description of Artificial Sequence: Maize gene-specific PCR primer
<400> 22
   cccagcacgc gtttccgacc tggatac 27
<210> 23
   <211> 26
   <212> DNA
   <213> Artificial Sequence
<220>
   <223> Description of Artificial Sequence: Maize gene-specific PCR primer
<400> 23
   tggccaataa ccacaatgtt gatgtg 26
<210> 24
   <211> 27
   <212> DNA
   <213> Artificial Sequence
<220>
   <223> Description of Artificial Sequence: Maize gene-specific PCR primer
<400> 24
   cgcttgtgca tcgagtcacg cgagata 27
<210> 25
   <211> 27
   <212> DNA
   <213> Artificial Sequence
<220>
   <223> Description of Artificial Sequence: Maize gene-specific PCR primer
<400> 25
   gaaggctgtg gaggagaggg ccatggt 27
<210> 26
   <211> 24
   <212> DNA
   <213> Artificial Sequence
<220>
   <223> Description of Artificial Sequence: Maize gene-specific PCR primer
<400> 26
   acccattgat cccgatcttg atct 24
<210> 27
   <211> 34
   <212> DNA
   <213> Artificial Sequence
<220>
   <223> Description of Artificial Sequence: Maize gene-specific PCR primer
<400> 27
   gaccatggtg tcgtgtggat ccgatgcggc tgct 34
<210> 28
   <211> 33
   <212> DNA
   <213> Artificial Sequence
<220>
   <223> Description of Artificial Sequence: Maize gene-specific PCR primer
<400> 28
   gaccatggtg tcgtgtggat ccccttgtgg tgc 33
<210> 29
   <211> 36
   <212> DNA
   <213> Artificial Sequence
<220>
   <223> Description of Artificial Sequence: Maize gene-specific PCR primer
<400> 29
   gaccatggtg tcgtgtggat ccggtgttgt tgaacg 36
<210> 30
   <211> 34
   <212> DNA
   <213> Artificial Sequence
<220>
   <223> Description of Artificial Sequence: Maize gene-specific PCR primer
<400> 30
   gaccatggtg tcgtgtggat ccgtgagatt gaac 34
<210> 31
   <211> 36
   <212> DNA
   <213> Artificial Sequence
<220>
   <223> Description of Artificial Sequence: Maize gene-specific PCR primer
<400> 31
   gaccatggtg tcgtcgtgtg gatccgtgaa gcttaa 36
<210> 32
   <211> 36
   <212> DNA
   <213> Artificial Sequence
<220>
   <223> Description of Artificial Sequence: Maize gene-specific PCR primer
<400> 32
   gaccatggtg tcgtgtggat ccgcctgctc cttgtc 36
<210> 33
   <211> 33
   <212> DNA
   <213> Artificial Sequence
<220>
   <223> Description of Artificial Sequence: Maize gene-specific PCR Primer
<400> 33
   gaccatggtg tcgtgtggat cctgcactgc tac 33
<210> 34
   <211> 34
   <212> DNA
   <213> Artificial Sequence
<220>
   <223> Description of Artificial Sequence: Maize gene-specific PCR primer
<400> 34
   gaccatggtg tcgtgtggat ccacaaacac aagc 34